## Europäisches Patentamt
## European Patent Office
## Office européen des brevets

(11) Veröffentlichungsnummer: **0 586 849 B1**

# (12) EUROPÄISCHE PATENTSCHRIFT

(45) Veröffentlichungstag der Patentschrift: **25.10.95**

(51) Int. Cl.6: **C09D 4/00**, C08F 220/36, C07C 219/08, C07C 217/50, C07C 229/12, C07C 229/16

(21) Anmeldenummer: **93111848.3**

(22) Anmeldetag: **23.07.93**

(54) **Aminoacrylate, ein Verfahren zu ihrer Herstellung und ihre Verwendung.**

(30) Priorität: **05.08.92 DE 4225921**

(43) Veröffentlichungstag der Anmeldung:
**16.03.94 Patentblatt 94/11**

(45) Bekanntmachung des Hinweises auf die
Patenterteilung:
**25.10.95 Patentblatt 95/43**

(84) Benannte Vertragsstaaten:
**AT BE CH DE DK ES FR GB IT LI NL SE**

(56) Entgegenhaltungen:
**EP-A- 0 231 442**
**DE-A- 3 706 355**
**DE-A- 3 836 370**

(73) Patentinhaber: **BAYER AG**

**D-51368 Leverkusen (DE)**

(72) Erfinder: **Meixner, Jürgen, Dr.**
**Josef-Brocker-Dyk 56**
**D-47803 Krefeld (DE)**
Erfinder: **Fischer, Wolfgang, Dr.**
**Eschendonk 6**
**D-40668 Meerbusch (DE)**
Erfinder: **Zwiener, Christian, Dr.**
**Hitdorfstrasse 9**
**D-50735 Köln (DE)**

**Beschreibung**

Die Erfindung betrifft neue Aminoacrylate und ein Verfahren zu ihrer Herstellung durch Addition von primären Mono- oder Diaminen mit aliphatisch gebundenen Aminogruppen an Acrylsäureester von ethoxylierten 3- oder 4-wertigen Alkoholen und ihre Verwendung als Bindemittel für strahlenhärtbare Überzugsmassen.

Es ist bekannt, daß durch die Addition von einem Unterschuß primärer oder sekundärer Amine an ungesättigte Polyacrylate, die analog einer Michael-Addition verläuft (Houben Weyl, Bd. 11/1, S. 277 ff., 1957), Verbindungen entstehen, die auch in Gegenwart von Luft durch Strahlenhärtung schnell und vollständig aushärten (DE-OS 23 46 424, DE-OS 37 06 355).

Während in der DE-OS 23 46 424 sehr allgemeine Angaben zum Aufbau solcher Aminoacrylate beschrieben sind, beschränkt sich die DE-OS 37 06 355 auf Aminoacrylate aus primären Monoaminen und Acrylsäureestern. Man findet jedoch in keiner der erwähnten Literaturstellen einen Hinweis auf niedrigviskose Produkte mit besonders hoher Reaktivität bei der Aushärtung mittels Strahlenhärtung.

Aufgabe der vorliegenden Erfindung war es, Produkte mit einer Viskosität (100 %ig, 23°C) von maximal 3000 mPa.s zur Verfügung zu stellen, die eine besonders schnelle Aushärtung bei Bestrahlung erlauben.

Diese Aufgabe konnte mit der Bereitstellung der nachstehend näher beschriebenen, erfindungsgemäßen Aminoacrylate bzw. des Verfahrens zu ihrer Herstellung gelöst werden Erfindungswesentlich ist die Beobachtung, daß zur Lösung der genannten Aufgabe als Reaktionspartner bei der Addition von primären Mono- oder Diaminen nur spezielle Acrylsäureester der nachstehend näher beschriebenen Art geeignet sind.

Gegenstand der Erfindung sind Aminoacrylate mit einer Viskosität bei 23°C von 200 bis 3000 mPa.s, einem Gehalt an olefinischen Doppelbindungen (berechnet als C = C, Molekulargewicht = 24) von 8 bis 20 Gew.-% und einem Stickstoffgehalt von 0,4 bis 2,0 Gew.-%, hergestellt durch Addition von

a) primären Mono- oder Diaminen des Molekulargewichtsbereichs 31 bis 300 mit aliphatisch gebundenen primären Aminogruppen an

b) Estergruppen aufweisende, unter Einhaltung eines COOH/OH-Äquivalentverhältnisses von 0,8:1 bis 1:1 hergestellte Umsetzungsprodukte von

b1) Acrylsäure mit

b2) mindestens eine Ethylenoxideinheit -CH$_2$-CH$_2$-O- als Teil einer Etherstruktur aufweisenden, 3- oder 4-wertigen Etheralkoholen des Molekulargewichtsbereichs 136 bis 1000, die im wesentlichen frei von Propylenoxideinheiten -CH$_2$-CH(CH$_3$)-O- sind.

Gegenstand der Erfindung ist auch ein Verfahren zur Herstellung derartiger Aminoacrylate durch Addition von

a) primären Mono- oder Diaminen des Molekulargewichtsbereichs 31 bis 300 mit aliphatisch gebundenen primären Aminogruppen an

b) Acrylsäureester mehrwertiger Alkohole unter Einhaltung eines Molverhältnisses von primären Aminogruppen der Komponente a) zu olefinischen Doppelbindungen der Komponente b) von 0,05:1 bis 0,25:1,

dadurch gekennzeichnet, daß man als Komponente b) unter Einhaltung eines Äquivalentverhältnisses von Carboxylzu Hydroxylgruppen von 0,8:1 bis 1:1 hergestellte, Estergruppen aufweisende Umsetzungsprodukte von

b1) Acrylsäure mit

b2) mindestens eine Ethylenoxideinheit -CH$_2$-CH$_2$-O- als Teil einer Etherstruktur aufweisenden 3- oder 4-wertigen Etheralkoholen verwendet, die im wesentlichen frei von Propylenoxideinheiten -CH$_2$-CH(CH$_3$)-O- sind.

Gegenstand der Erfindung ist schließlich auch die Verwendung der neuen Aminoacrylate als Bindemittel für strahlenhärtbare Überzugsmassen.

Bei der Komponente a) handelt es sich um primäre Mono- oder Diamine des Molekulargewichtsbereichs 31 bis 300, vorzugsweise 45 bis 250 mit aliphatisch gebundenen primären Aminogruppen. Die Monoamine sind gegenüber den Diaminen bevorzugt. In Betracht kommen Monoamine wie Methylamin, n-Butylamin, n-Hexylamin, 2-Ethylhexylamin, Cyclohexylamin, Ethanolamin oder Benzylamin oder auch Diamine wie Ethylendiamin, die isomeren Diaminobutane, die isomeren Diaminohexane oder 1,4-Diaminocyclohexan.

Bei der Komponente b) handelt es sich um Estergruppen aufweisende Umsetzungsprodukte aus b1) Acrylsäure und b2) 3- und/oder 4-wertigen, Ethergruppen aufweisenden Alkoholen. Die Umsetzung der Komponenten b1) und b2) erfolgt unter Einhaltung eines Molverhältnisses von Carboxylgruppen zu Hydroxylgruppen von 0,8:1 bis 1:1.

2

Bei den erfindungswesentlichen mehrwertigen Alkoholen b2) handelt es sich um solche des Molekulargewichtsbereichs 136 bis 1000, die mindestens eine Ethylenoxideinheit $-CH_2-CH_2-O-$ als Teil einer Etherstruktur und 3 oder 4 alkoholische Hydroxylgruppen aufweisen oder um deren Gemische. Derartige Etheralkohole werden durch Ethoxylierung von geeigneten Startermolekülen in an sich bekannter Weise erhalten. Geeignete Startermoleküle sind insbesondere die den Etheralkoholen entsprechenden Ethergruppen-freien 3- oder 4-wertigen Alkohole wie beispielsweise Glycerin, Trimethylolpropan, Trimethylolethan, Pentaerythrit oder beliebige Gemische derartiger mehrwertiger Alkohole. Die zum Einsatz gelangenden Etheralkohole weisen im allgemeinen einen Ethoxylierungsgrad von 1 bis 10, vorzugsweise von 2 bis 8 auf, wobei der Ethoxylierungsgrad die Anzahl der Mole Ethylenoxid angibt, die an 1 Mol eines als Startermolekül verwendeten Alkohols im Durchschnitt angelagert worden sind Besonders bevorzugt werden ethoxylierte 3- oder 4-wertige Alkohole mit einem Ethoxylierungsgrad von 3 bis 6 und einem Molekulargewicht von 224 bis 400 eingesetzt.

Die Ethergruppen aufweisenden Alkohole b2) sind im wesentlichen frei von Propylenoxideinheiten $-CH_2-CH(CH_3)-O-$. Dies bedeutet, daß bei der Alkoxylierungsreaktion neben Ethylenoxid allenfalls untergeordnete Mengen an Propylenoxid mitverwendet werden (Molverhältnis von Ethylenoxid zu Propylenoxid mindestens 5:1), da sonst die erfindungsgemäß angestrebten Eigenschaften der Aminoacrylate nicht erreicht werden können Besonders bevorzugt handelt es sich bei der Komponente b2) ausschließlich um Ethoxylierungsprodukte der beispielhaft genannten Startermoleküle.

Die Estergruppen aufweisenden Umsetzungsprodukte b) weisen vorzugsweise einen Gehalt an olefinischen Doppelbindungen (berechnet als $C = C$, Molekulargewicht $= 24$) von mindestens 10 Gew.-% auf.

Die erfindungsgemäße Umsetzung zwischen den Ausgangskomponenten a) und b) kann in Substanz oder auch in Gegenwart von geeigneten Lösungsmitteln wie beispielsweise Ethylacetat und/oder Toluol erfolgen. Die Reaktionstemperatur liegt im allgemeinen bei 10 bis 100, vorzugsweise 20 bis 60°C. Zur Durchführung der erfindungsgemäßen Umsetzung kann man beispielsweise so vorgehen, daß man das, gegebenenfalls in einem inerten Lösungsmittel der genannten Art gelöste, Estergruppen aufweisende Umsetzungsprodukt b) vorlegt und anschließend unter Rühren die Aminkomponente a) zugibt, um schließlich das Reaktionsgemisch so lange zu rühren bis keine Wärmetönung durch exotherme Additionsreaktion mehr erkennbar ist, Im Falle der Mitverwendung von inerten Lösungsmitteln können diese gewünschtenfalls im Anschluß an die Reaktion destillativ entfernt werden. Die Menge der zugesetzten Aminkomponente a) wird dabei so bemessen, daß ein Stickstoffgehalt im Reaktionsprodukt von 0,4 bis 2,0 Gew.-% resultiert, was im allgemeinen einem Molverhältnis von Aminogruppen der Komponente a) zu olefinischen Doppelbindungen der Komponente b) von 0,05:1 bis 0,25:1 entspricht. Die auf diese Weise hergestellten Umsetzungsprodukte weisen einen Gehalt an olefinischen Doppelbindungen (berechnet als $C = C$, Molekulargewicht $= 24$) von 8 bis 20, vorzugsweise 10 bis 18 Gew.-% und eine Viskosität bei 23°C von 200 bis 3000, vorzugsweise 300 bis 2000 mPa.s auf.

Erforderlichenfalls kann bei der Durchführung der erfindungsgemäßen Umsetzung zur Verhinderung einer unerwünschten Polymerisation dem Reaktionsgemisch ein oder mehrere Polymerisationsinhibitoren zugesetzt werden. Geeignet sind beispielsweise Phenole wie 2,6-Di-tert.-butyl-p-kresol, Hydrochinone wie die isomeren Methylhydrochinone oder Phenothiazin.

Die erfindungsgemäßen Aminoacrylate stellen wertvolle Bindemittel für strahlenhärtbare Überzugsmassen dar. Im Falle der Verwendung in UV-härtbaren Systemen müssen diesen Systemen Fotoinitiatoren zugesetzt werden. Brauchbare Fotoinitiatoren sind z.B. Benzophenon, Benzoin und Benzoinether sowie Benzilketale und Hydroxyalkylphenone. Diese Substanzen werden im allgemeinen in Konzentrationen von 0,1 bis 5 Gew.-% zugesetzt.

Grundsätzlich können auch andere ionisierende Strahlungsarten wie Elektronenstrahlen zur Anwendung gelangen.

In den nachfolgenden Beispielen beziehen sich alle Prozentangaben auf Gewichtsprozente.

Beispiele

Acrylsäureester b)

Die in Tabelle 1 genannte Menge an Alkohol wird zusammen mit der genannten Menge an Acrylsäure sowie 1,5 % p-Toluolsulfonsäure als Katalysator und 0,1 % p-Methoxyphenol und 0,1 % 3,4-Di-tert.-butylhydrochinon als Inhibitoren, stets bezogen auf die Summe von Etheralkohol und Acrylsäure, 70 %ig in Toluol gelöst. Das Gemisch wird unter Durchleiten von Luft solange auf 100 bis 120°C unter fort laufender Entfernung des entstehenden Wassers durch azeotrope Destillation erhitzt, bis sich kein Wasser mehr abscheidet. Nach Abkühlen wird bei 50 bis 90°C das Lösungsmittel unter Vakuum abdestilliert.

## Tabelle 1

| Acrylsäureester b) | ba | bb | bc | bd | be | bf | bg |
|---|---|---|---|---|---|---|---|
| Alkohol b2) (Mol) | | | | | | | |
| 3-fach ethoxyliertes Glycerin | 1,0 | – | – | – | – | – | – |
| 4-fach ethoxyliertes Trimethylolpropan | – | 1,0 | – | 1,0 | – | – | – |
| 4-fach ethoxyliertes Pentaerythrit | – | – | 1,0 | – | – | – | – |
| 12-fach ethoxyliertes Trimethylolpropan | – | – | – | – | 1,0 | – | – |
| 3-fach propoxyliertes Trimethylolpropan | – | – | – | – | – | 1,0 | – |
| Trimethylolpropan | – | – | – | – | – | – | 1,0 |
| Acrylsäure b1) (Mol) | 2,5 | 2,5 | 3,7 | 2,3 | 2,5 | 3,0 | 3,0 |
| COOH/OH-Verhältnis | 0,83:1 | 0,83:1 | 0,92:1 | 0,76:1 | 0,83:1 | 1:1 | 1:1 |
| Viskosität (mPa.s/23°C) | 150 | 160 | 140 | 170 | 200 | 90 | 100 |

Aminoacrylate

Der in Tabelle 2 genannten Menge an Acrylsäureester b) wird bei 60°C die in Tabelle 2 genannte Menge an Amin a) zugegeben. Die Mischung wird bei 60°C 5 Stunden gehalten und dann abgekühlt.
Beispiele 1 bis 5 sind erfindungsgemäß.

In Vergleichsbeispiel 6 wird Acrylsäureester bd) eingesetzt, der weniger als die erfindungsgemäße Menge an Carboxyläquivalent pro Hydroxyäquivalent enthält.

In Vergleichsbeispiel 7 sind weniger als die erfindungsgemäße Menge an olefinischen Doppelbindungen enthalten.

In Vergleichsbeispiel 8 wird mit einem propoxylierten Etheralkohol bf) statt einem ethoxylierten Etheralkohol gearbeitet.

Vergleichsbeispiel 9 enthält einen Alkohol bg) ohne Etherstrukturen.

Vergleichsbeispiel 10

Das Beispiel 2 der DE-OS 37 06 355 wird nachgestellt.

Es handelt sich dabei um ein Addukt aus Ethanolamin an ein Polyetheracrylat, das durch Umsetzung von Trimethylolpropan mit Ethylenoxid und Propylenoxid (Mol-Verhältnis Ethylenoxid : Propylenoxid = 1:2,5) sowie anschließende Umsetzung mit Acrylsäure erhalten wurde.

Viskosität (23°C/mPa.s): 550 mPa.s

= C = C = Doppelbindungsgehalt: 12,0 %

Stickstoffgehalt: 0,24 %

Tabelle 2

| Ausgangsmaterialien (%) | erfindungsgemäße Beispiele | | | | | Vergleichsbeispiele | | | |
|---|---|---|---|---|---|---|---|---|---|
| | 1 | 2 | 3 | 4 | 5 | 6 | 7 | 8 | 9 |
| Acrylsäureester | | | | | | | | | |
| ba | 97,5 | – | – | – | – | – | – | – | – |
| bb | – | 97,4 | 96,6 | – | 94,6 | – | – | – | – |
| bc | – | – | – | 96,6 | – | – | – | – | – |
| bd | – | – | – | – | – | 96,6 | – | – | – |
| bg | – | – | – | – | – | – | 97,5 | – | – |
| be | – | – | – | – | – | – | – | 96,6 | – |
| bf | – | – | – | – | – | – | – | – | 96,6 |
| Ethanolamin | 2,5 | 2,6 | 3,4 | 3,4 | – | 3,4 | 2,5 | 3,4 | 3,4 |
| Cyclohexylamin | | | | | 5,4 | | | | |
| Viskosität (mPa.s/23° C) | 550 | 440 | 780 | 630 | 570 | 1400 | 1700 | 520 | 540 |
| Olefinische Doppelbindungen (%) (MG = 24) | 16,3 | 13,2 | 13,1 | 15,3 | 12,9 | 12,4 | 7,3 | 14,8 | 23,5 |
| Stickstoffgehalt (%) | 0,6 | 0,6 | 0,8 | 0,8 | 0,8 | 0,8 | 0,6 | 0,8 | 0,8 |

Verwendungsbeispiele

Die Aminoacrylate der Beispiele 1 bis 5 sowie der Vergleichsbeispiele 6 bis 10 werden mit je 6,5 Teilen ®Esacure TZT (Trimethylbenzophenon, Isomerengemisch, Handelsprodukt der Firma Fratelli Lamberti)

EP 0 586 849 B1

versetzt. Nach Auftragen der Lackfilme auf Karton (250 g/m$^2$) werden diese unter einem Hanoviastrahler (80 W/cm, 10 cm Abstand) durchbewegt. Bei 70 m/min Bandgeschwindigkeit entstehen nur bei den erfindungsgemäßen Beispielen lösemittelfeste Überzüge. Die Lackfilme der Vergleichsbeispiele bleiben klebrig.

Lösemittelfest bedeutet in diesem Fall, daß der Lackfilm nach mindestens 20 Doppelhüben mit einem in Butylacetat getränkten Tuch unter 1 kg Belastung noch einwandfrei aussieht.

**Patentansprüche**

1.  Aminoacrylate mit einer Viskosität bei 23°C von 200 bis 3000 mPa.s, einem Gehalt an olefinischen Doppelbindungen (berechnet als C=C, Molekulargewicht = 24) von 8 bis 20 Gew.-% und einem Stickstoffgehalt von 0,4 bis 2,0 Gew.-%, hergestellt durch Addition von
    a) primären Mono- oder Diaminen des Molekulargewichtsbereichs 31 bis 300 mit aliphatisch gebundenen primären Aminogruppen an
    b) Estergruppen aufweisende, unter Einhaltung eines COOH/OH-Äquivalentverhältnisses von 0,8:1 bis 1:1 hergestellte Umsetzungsprodukte von
    b1) Acrylsäure mit
    b2) mindestens eine Ethylenoxideinheit -CH$_2$-CH$_2$-O- als Teil einer Etherstruktur aufweisenden, 3- oder 4-wertigen Etheralkoholen des Molekulargewichtsbereichs 136 bis 1000, die im wesentlichen frei von Propylenoxideinheiten -CH$_2$-CH(CH$_3$)-O- sind.

2.  Verfahren zur Herstellung von Aminoacrylaten gemäß Anspruch 1 durch Addition von
    a) primären Mono- oder Diaminen des Molekulargewichtsbereichs 31 bis 300 mit aliphatisch gebundenen primären Aminogruppen an
    b) Acrylsäureester mehrwertiger Alkohole unter Einhaltung eines Molverhältnisses von primären Aminogruppen der Komponente a) zu olefinischen Doppelbindungen der Komponente b) von 0,05:1 bis 0,25:1,
    dadurch gekennzeichnet, daß man als Komponente b) unter Einhaltung eines Äquivalentverhältnisses von Carboxyl- zu Hydroxylgruppen von 0,8:1 bis 1:1 hergestellte, Estergruppen aufweisende Umsetzungsprodukte von
    b1) Acrylsäure mit
    b2) mindestens eine Ethylenoxideinheit -CH$_2$-CH$_2$-O- als Teil einer Etherstruktur aufweisenden 3- oder 4-wertigen Etheralkoholen verwendet, die im wesentlichen frei von Propylenoxideinheiten -CH$_2$-CH(CH$_3$)-O- sind.

3.  Verwendung der Aminoacrylate der gemäß Anspruch 1 als Bindemittel für strahlenhärtbare Überzugsmassen.

**Claims**

1.  Aminoacrylates with a viscosity of 200 to 3000 mPa.s at 23°C, a proportion of olefinic double bonds (calculated as C=C), molecular weight = 24) of 8 to 20% by weight and a nitrogen content of 0.4 to 2.0% by weight, prepared by the addition of
    a) primary mono- or diamines with molecular weights in the range 31 to 300 with aliphatically bonded primary amino groups to
    b) reaction products which possess ester groups prepared, while maintaining a COOH/OH ratio by equivalents of 0.8:1 to 1:1, from
    b1) acrylic acid and
    b2) 3- or 4-hydric ether-alcohols with molecular weights in the range 136 to 1000 which possess at least one ethylene oxide unit -CH$_2$-CH$_2$-O- as part of an ether structure, which are in principle free of propylene oxide units -CH$_2$-CH(CH$_3$)-O-.

2.  A process for preparing aminoacrylates according to Claim 1 by the addition of
    a) primary mono- or diamines with molecular weights in the range 31 to 300 with aliphatically bonded primary amino groups to
    b) acrylic esters of polyhydric alcohols while maintaining a molar ratio of primary amino groups from component a) to olefinic double bonds from component b) of 0.05:1 to 0.25:1,
    characterised in that reaction products possessing ester groups are prepared, while maintaining a ratio by equivalents of carboxyl to hydroxyl groups of 0.8:1 to 1:1, from

7

b1) acrylic acid and

b2) 3- or 4-hydric ether-alcohols which possess at least one ethylene oxide unit $-CH_2-CH_2-O-$ as part of an ether structure, which are in principle free of propylene oxide units $-CH_2-CH(CH_3)-O-$, are used as component b).

3. Use of aminoacrylates according to Claim 1 as binders for coating materials which can be radiation cured.

**Revendications**

1. Aminoacrylates présentant une viscosité à 23° C de 200 à 3000 mPa.s, une teneur en liaisons doubles oléfiniques (comptées C = C, poids moléculaire = 24) de 8 à 20 % en poids et une teneur en azote de 0,4 à 2,0 % en poids, préparés par addition de :

a) monoamines ou diamines primaires de poids moléculaire compris entre 31 et 300 comportant des groupes amino primaires liés à des radicaux aliphatiques à

b) des produits de transformation comportant des groupes ester et préparés en maintenant un rapport d'équivalence entre COOH et OH de 0,8:1 à 1:1, à partir

b1) d'acide acrylique et

b2) d'étheralcools trivalents ou tétravalents de poids moléculaire compris entre 136 et 1000, présentant au moins une unité d'oxyde d'éthylène $-CH_2-CH_2-O-$ faisant partie d'une structure éther, qui sont essentiellement dépourvus d'unités d'oxyde de propylène $-CH_2-CH(CH_3)-O-$.

2. Procédé de préparation d'aminoacrylates selon la revendication 1 par addition de :

a) monoamines ou diamines primaires de poids moléculaire compris entre 31 et 300, comportant des groupes amino primaires liés à des radicaux aliphatiques à

b) des acrylates d'alcools polyvalents, en maintenant un rapport molaire entre groupes amino primaires du composant a) et liaisons doubles oléfiniques du composant b) allant de 0,05:1 à 0,25:1, caractérisé en ce que l'on utilise comme composant b) des produits de transformation comportant des groupes ester, préparés en maintenant un rapport d'équivalence entre groupes carboxyle et groupes hydroxyle allant de 0,8:1 à 1:1 à partir

b1) d'acide acrylique et

b2) d'étheralcools trivalents ou tétravalents, présentant au moins une unité d'oxyde d'éthylène $-CH_2-CH_2-O-$ faisant partie d'une structure éther, qui sont essentiellement dépourvus d'unités d'oxyde de propylène $-CH_2-CH(CH_3)-O-$.

3. Utilisation des aminoacrylates selon la revendication 1 en tant qu'agents liants pour les couches de revêtement durcissables par rayonnement.